# EUROPÄISCHE PATENTSCHRIFT

(11) **EP 1 538 443 B1**
(45) Veröffentlichungstag und Bekanntmachung des Hinweises auf die Patenterteilung: **21.07.2010**
(21) Anmeldenummer: 04028338.4
(22) Anmeldetag: 30.11.2004
(51) Int. Cl.: G01N 33/487

(54) **Schnelldiagnosegerät mit Verriegelung durch Teststreifen**
Rapid diagnosis apparatus comprising locking means actuated by test strips
Appareil pour un diagnostic rapid comportant des moyens de verrouillage actionnes par des bandes d'essai

(30) Priorität: 03.12.2003 DE 10356452
(43) Veröffentlichungstag der Anmeldung: 08.06.2005
(73) Patentinhaber: F.HOFFMANN-LA ROCHE AG, 4070 Basel (CH); Roche Diagnostics GmbH, 68305 Mannheim (DE)
(72) Erfinder: Augstein, Manfred, 68305 Mannheim (DE); Riebel, Stefan, 76756 Bellheim (DE); Stöhr, Rainer, 96524 Föritz (DE)
(74) Vertreter: Isenbruck, Günter

(56) Entgegenhaltungen:
- EP-A- 0 492 326
- WO-A-02/094092
- US-A- 5 304 468
- US-A- 5 515 170

## Beschreibung

Die Erfindung bezieht sich auf ein Schnelldiagnosegerät mit Verriegelung eines Deckelelementes insbesondere bei in das Schnelldiagnosegerät eingeführtem Teststreifen, wobei das Schnelldiagnosegerät ein Heizelement besitzt und Umwelteinflüssen ausgesetzt ist.

### Stand der Technik

Bei Schnelldiagnosegeräten, wie sie heute üblicherweise zur Auswertung von Teststreifen eingesetzt werden, kommt es bei längerem Betrieb zur Verschmutzung eines im Schnelldiagnosegerät eingesetzten Heizelementes. Die Anordnung des Heizelementes ist erforderlich, um den Teststreifen auf eine bestimmte, eine optimale Auswertung der Messprobe ermöglichende Temperatur zu bringen. Am Heizelement, welches innerhalb des Gerätegehäuses des Schnelldiagnosegerätes aufgenommen ist, lagern sich im Laufe der Zeit Partikel ab, die von Zeit zu Zeit entfernt werden müssen, da bei zunehmenden Partikelaufbau am Heizelement dessen Wirkungsgrad beeinträchtigt bzw. die Stromaufnahme des Heizelementes zu groß wird. Um das innerhalb des Gerätegehäuses des Schnelldiagnosegerätes aufgenommene Heizelement von Zeit zu Zeit zu reinigen, ist die Entfernung eines Deckel- oder eines Klappenelementes, welches in das Gerätegehäuse eingelassen ist, erforderlich. Daher ist das Gerätegehäuse mit einer im allgemeinen als Deckel oder als Klappe ausgestalteten, aus dem Gerätegehäuse entnehmbaren Flächenelement versehen. Durch das entnehmbare Flächenelement kann zudem störendes Umlicht in das Innere des Gerätegehäuses einfallen, was insbesondere bei optischen Messgeräten, mit denen Testelemente oder Teststreifen auf optischem Wege ausgewertet werden, die Messung erheblich beeinträchtigen kann.

Ein Schnelldiagnosegerät mit Klappdeckel ist in EP-A-0 492 326 beschrieben.

Im Allgemeinen liegt eine durch den Deckel bzw. ein Klappenelement verschließbare Gehäuseöffnung an der Seite des Gerätegehäuses des Schnelldiagnosegerätes, in welchem die Einschuböffnung für einen in das Gerätegehäuseinnere einzuschiebenden Teststreifen liegt.

Es hat sich herausgestellt, dass es beim Einschieben des innerhalb des Schnelldiagnosegerätes auszuwertenden Teststreifens vereinzelt dazu kommt, dass der das Gerätegehäuse verschließende Deckel bzw. die Klappe versehentlich oder absichtlich geöffnet wird, was zu Fehlmessungen, d.h. fehlerhaften Auswertungen des in das Gehäuseinnere eingeschobenen Teststreifens führen kann.

Die Fehlmessungen rühren daher, dass es aufgrund der Abnahme des Deckels bzw. Öffnen der Klappe an der Vorderseite des Gerätegehäuses des Schnelldiagnosegerätes zu einer plötzlichen Temperaturänderung in der Umgebung des auszuwertenden Teststreifens kommen kann. Das fehlende Deckelelement bzw. das fehlende Klappenelement an der Gehäuseöffnung erlaubt einen Zutritt von Umgebungsluft, welche das durch das Heizelement im Inneren des Gerätegehäuses erzeugte Temperaturniveau erheblich beeinflusst, so dass ein unter wechselnden Umgebungsbedingungen hinsichtlich der Temperatur ausgewerteter Teststreifen zu einem unbrauchbaren Testergebnis führen kann. Bei fehlendem Deckelelement kann zudem störendes Umlicht insbesondere bei optischen Messgeräten in das Innere des Gerätegehäuses einfallen, was die Auswertung optisch auszuwertender Testelemente erheblich beeinträchtigt.

Neben einer fehlerhaften Messung kann es darüber hinaus zum Abbruch der Messung kommen.

Angesichts der vorstehend skizzierten Nachteile von Schnelldiagnosegeräten, in welchen ein Teststreifen ausgewertet wird, liegt der Erfindung die Aufgabe zugrunde, das Schnelldiagnosemessgerät während der Auswertung eines Teststreifens gegen ein versehentliches oder unbeabsichtigtes Öffnen abzusichern und störende Umwelteinflüsse vom Inneren des Gerätes fernzuhalten.

Erfindungsgemäß wird die Aufgabe durch die Merkmale des Patentanspruches 1 gelöst.

Durch die erfindungsgemäß vorgeschlagene. Lösung wird ein Schnelldiagnosegerät zur Verfügung gestellt, dessen Gehäuse gegen ein unbeabsichtigtes Öffnen während eines Messvorganges gesichert ist. Dazu wird erfindungsgemäß der zur Auswertung in eine Einschuböffnung eingeschobene Teststreifen herangezogen, der ein Bügelteil eines Federelementes derart auslenkt, dass eine zum Öffnen des Deckel- oder Klappenelementes am Gehäuse erforderliche Entriegelung des Deckelelementes blockiert ist. Die erfindungsgemäß vorgeschlagene Verriegelung durch den in eine Einschuböffnung eingeschobenen, innerhalb des Schnelldiagnosegerätes auszumessenden Teststreifens, erfolgt unter Einsatz eines Federelementes, welches an die Unterseite des die Gehäuseöffnung verschließenden Deckel- oder Klappenelementes eingeklippst ist.

Das an die Unterseite des Deckel- oder Klappenelementes eingeklippsbare Federelement umfasst in vorteilhafter Weise einen in eine Federzunge übergehenden Bügelabschnitt sowie zwei Federschenkel. An den Federschenkeln können im Bereich von Anschlägen diesen gegenüberliegende Zapfenelemente ausgebildet sein, mit deren Hilfe das Federelement in entsprechende Aufnahmeöffnungen an der Unterseite des Deckel- oder Klappenelementes einklippsbar ist. Das Federelement kann alternativ auch im Gerätegehäuse sitzen.

Das Bügelelement, welches sich im wesentlichen zwischen den beiden Schenkeln des Federelementes erstreckt, kann eine gekröpfte Ausbildung haben sowie an seiner dem Teststreifen zuweisenden Seiten Anlagestege aufweisen, mit welchen die Einschubbewegung des Teststreifens in das Gehäuseinnere des Schnelldiagnosegerätes unterstützt werden kann.

Durch die erfindungsgemäße Lösung wird erreicht, dass bei in einem Aufnahmeschlitz einsteckenden Teststreifen, der zwischen den beiden Federschenkeln liegende Bügelabschnitt so ausgelenkt ist, dass eine seitliche Entriegelungsbewegung der Anschläge an den beiden parallel zueinander verlaufenden Federschenkeln des Federelementes an der Unterseite des Deckel- oder Klappenelementes blockiert ist. Ist der Teststreifen hingegen nicht in den Einführschlitz eingeführt, so ragt der zwischen den beiden Federschenkeln angeordnete Bügelabschnitt in den Bereich hinein, in dem ansonsten der Teststreifen eingeführt ist. Aufgrund dessen liegen die beiden Anschläge, die an den beiden Federschenkeln einander gegenüberliegend angeordnet sind, nicht am Bügelabschnitt an und' vermögen eine seitliche Entriegelungsbewegung auszuführen, so dass das Deckelelement aus der Gehäuseöffnung des Gehäusekörpers des Schnelldiagnosemessgerätes entnehmbar ist.

### Zeichnungen

Anhand der Zeichnungen wird die Erfindung nachstehend eingehender beschrieben.

Es zeigt:
- Figur 1: die Draufsicht auf ein Gerätegehäuse eines Schnelldiagnosegerätes,
- Figur 2: die Unterseite eines eine Gerätegehäuseöffnung verschließenden Deckelelementes mit Verriegelungskörper,
- Figur 3: eine im vergrößertem Maßstab dargestellte Wiedergabe der Vorderkante des Gerätegehäuses mit freiliegender Gerätegehäuseöffnung,
- Figur 4: einen Bügelabschnitt des Verriegelungskörpers in einer durch einen eingeführten Teststreifen herbeigeführten Verriegelungsposition und
- Figur 5: den Bügelabschnitt des Verriegelungskörpers in einer ein entriegeltes Deckel- bzw. Klappenelementes ermöglichenden Position.

### Ausführungsvarianten

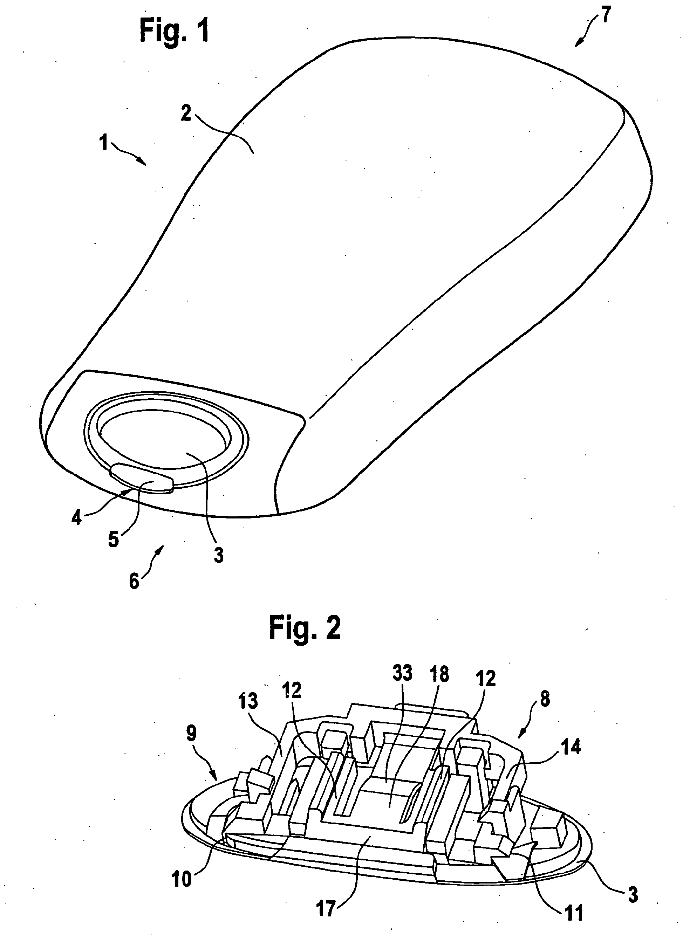

Aus der Darstellung gemäß Figur 1 geht in perspektivischer Wiedergabe ein Gerätegehäuse eines Schnelldiagnosegerätes hervor.

Ein in Figur 1 beispielhaft dargestelltes Schnelldiagnosegerät 1 umfasst ein Gerätegehäuse 2. Eine im Gerätegehäuse 2 ausgebildete Gerätegehäuseöffming ist durch ein Deckel- oder Klappenelement verschlossen. Unterhalb des Deckel- oder Klappenelementes 3 befindet sich eine schlitzförmig ausgeführte Einschuböffnung 4, in welcher ein innerhalb des Schnelldiagnosegerätes 1 auszuwertender Teststreifen einschiebbar ist. Die Einschuböffnung 4 für den Teststreifen wird einerseits durch einen Abschnitt des Deckeloder Klappenelementes 3 oder andererseits durch eine flach ausgebildete Einschubzunge 5 begrenzt. Die Einschuböffnung 4 für den in das Gerätegehäuse 2 einzuschiebenden Teststreifen befindet sich an einer Vorderseite 6 des Gerätegehäuses 2, dessen Rückseite mit Bezugszeichen 7 identifiziert ist. Ebenso gut könnte die Einschuböffnung 4, die gemäß der Ausführungsvariante des Schnelldiagnosegerätes 1 gemäß Figur 1 an dessen Vorderseite 6 ausgebildet ist, auch an dessen Rückseite 7 ausgebildet sein.

Figur 2 zeigt die Unterseite eines die Gehäuseöffnung verschließenden Deckel- oder Klappenelementes mit an diesem angebrachten Verriegelungskörper.

Aus der Ansicht gemäß Figur 2 geht hervor, dass an einer Deckel- oder Klappenelementunterseite 9 ein als Verriegelungskörper 8 ausgebildeter Verriegelungskörper aufgenommen ist. Dieser kann alternativ auch im Gerätegehäuse 2 sitzen. Am als Federelement ausgebildeten Verriegelungskörper 8 sind ein erster Federschenkel 13 und ein zweiter Federschenkel 14 ausgebildet. Die beiden Federschenkel 13 bzw. 14 erstrecken sich parallel zu einer Federzunge 18, an deren Ende ein Bügelabschnitt 17 ausgebildet ist. Die Federzunge 18 kann mit einer Kröpfung 33 versehen sein. Im durch die Kröpfung 33 nach oben vorstehenden Bereich des in Figur 2 dargestellten als Verriegelungskörper dienenden Federelementes 8 werden bevorzugt ein oder mehrere Anlagestege 12 zur Kontaktierung des Teststreifens ausgebildet.

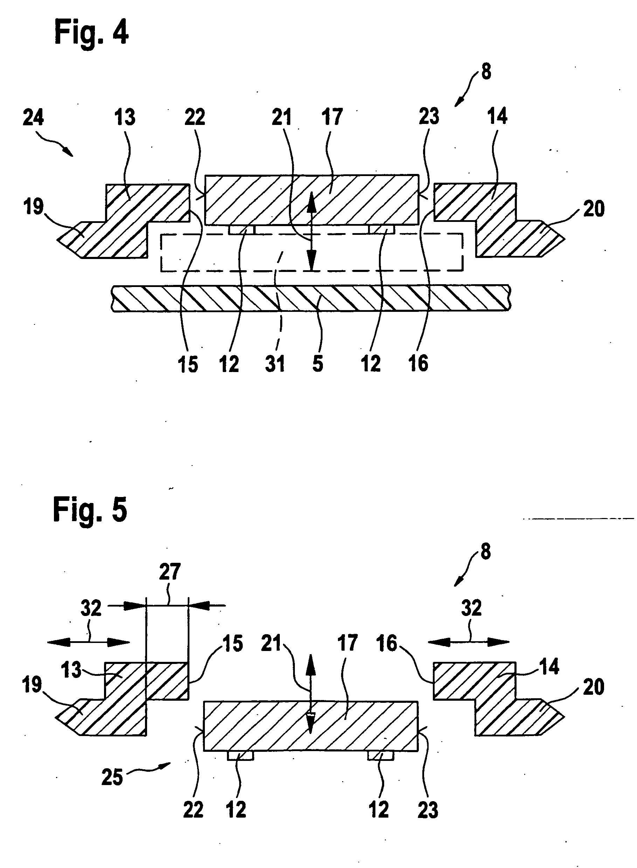

An den beiden Federschenkeln 13 bzw. 14 des als Verriegelungskörper dienenden Federelementes 8 können (vergleiche Darstellung gemäß der Figuren 4 und 5) Zapfen 19 und 20 angeordnet werden. Das als Verriegelungskörper eingesetzte Federelement 8 wird bevorzugt als Kunststoffspritzgießbauteil gemäß des Ein- oder Mehrkomponentenspritzgießverfahrens gefertigt, wodurch dieses einstückig ausgebildet werden kann und in einem Arbeitsgang sowohl die mit einer Kröpfung 33 versehene Federzunge 18 als auch die darin ausgebildeten Anlagestege 12, der erste Federschenkel 13 sowie der zweite Federschenkel 14 und an diese vorzugsweise angespritzte Zapfen 19 und 20 spritzgegossen werden.

Die in den Figuren 4 und 5 dargestellten Zapfen 19 und 20 werden in eine erste Einklippsstelle 10 sowie eine zweite Einklippsstelle 11 an der Deckelunterseite 9 des Deckel- oder Klapperielementes 3 eingeklippst.

Das in Figur 2 dargestellte, als Verriegelungskörper dienende Verriegelungskörper 8 ist von seiner Unterseite her gezeigt, mit welcher es einen in die Einschuböffnung 4 gemäß Figur 1 eingeschobenen Teststreifen gegenüberliegt.

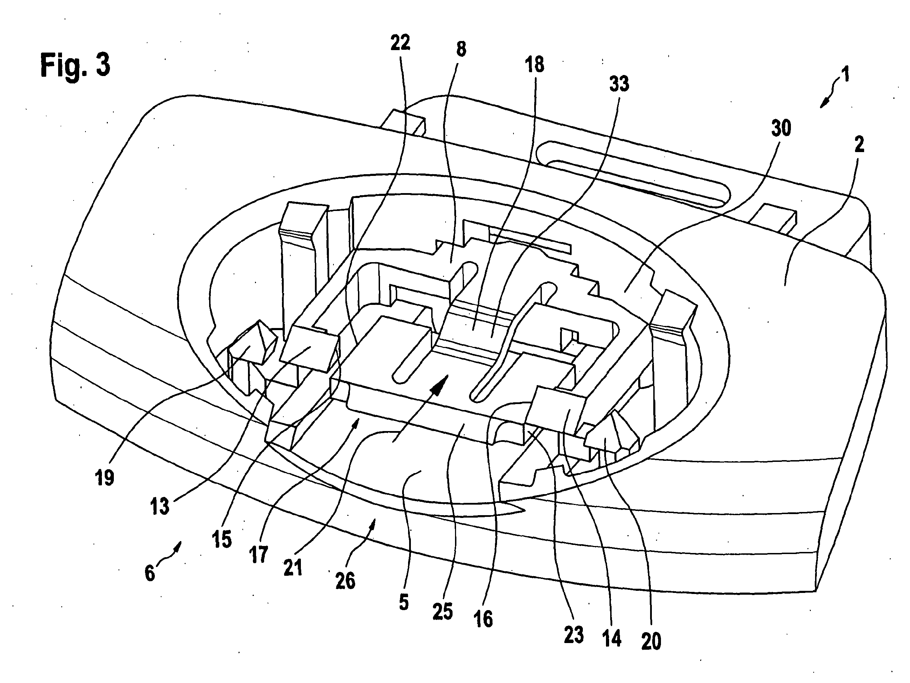

Aus der Darstellung gemäß Figur 3 geht die freiliegende Gehäuseöffnung an der Vorderkante des Gerätegehäuses des Schnelldiagnosegerätes in einem vergrößerten Maßstab wiedergegeben, hervor.

An der Vorderseite 6 des Gerätegehäuses 2 des Schnelldiagnosegerätes 1 ist eine oval ausgebildete Gehäuseöffnung 30 ausgebildet. Die Unterseite der Gehäuseöffnung 30 wird durch die Einschubzunge 5 für einen in Figur 3 nicht dargestellten Teststreifen gebildet. Oberhalb der Einführzunge 5 befindet sich der Bügelabschnitt 17 der Federzunge 18 in einer zweiten Bügelabschnittlage 25, in welcher eine Freigabe des aus der Gehäuseöffnung 30 entnommenen Deckel- bzw. Klappenelementes 3 ermöglicht wird. Aus der Darstellung gemäß Figur 3 geht hervor, dass die Federzunge 18, deren vorderes Ende durch den Bügelabschnitt 17 gebildet ist, eine Kröpfung 33 aufweist. Die Kröpfung 33 der Federzunge 18 verbessert die Anfederungseigenschaft der Federzunge 18 und den daran ausgebildete Anlagestegen 12 (vergleiche Darstellung gemäß Figur 2) an den in die Einschuböffnung 4 einzuschiebenden Teststreifen. An den sich beidseits der Federzunge 18 erstreckenden Federschenkeln 13 bzw. 14 sind jeweils Anschläge 15, 16 ausgebildet. Die Anschläge 15, 16 weisen Seitenflächen 22, 23 der Federzunge 18 zu, die in die durch den Pfeil 21 angedeutete Bewegungsrichtung ausgelenkt werden, sobald ein Teststreifen in die Einschuböffnung 4 eingeschoben wird.

In der in Figur 3 dargestellten zweiten Bügellage 25 des Bügelabschnittes 17 der Federzunge 18 liegt der Bügelabschnitt 17 unterhalb der einander zuweisenden Anschläge 15, 16 der Federschenkel 13 und 14. Dadurch wird deren seitliche Auslenkung (vergleiche Darstellung gemäß Figur 5) zur Entriegelung des Deckel- bzw. Klappenelementes 3 zu dessen Entnahme aus der Gehäuseöffnung 30 ermöglicht.

An den Außenseiten der parallel zueinander angeordneten Federschenkel 13 und 14 ist jeweils ein erster Zapfen 19 bzw. ein zweiter Zapfen 20 angespritzt, mit welchem das als Verriegelungskörper dienende Verriegelungskörper 8 in entsprechende Einklippsstellen 10, 11 an der Unterseite 9 des Deckel- bzw. Klappenelementes eingeklippst werden kann (vergleiche Darstellung gemäß Figur 2).

Der Darstellung gemäß Figur 4 ist ein durch einen Teststreifen ausgelenkter Bügelabschnitt des Federelementes zu entnehmen.

Aus der Darstellung gemäß Figur 4 geht hervor, dass ein Teststreifen 31 in die Einschuböffnung 4 im unteren Bereich der Gehäuseöffnung 30 an der Vorderseite 6 des Gerätegehäuses 2 eingeschoben ist. Beim Einschieben des Teststreifens 31 liegt dieser mit seiner Unterseite auf der Einschubzunge 5 (vergleiche Darstellung gemäß Figur 3) auf. Die Oberseite des Teststreifens 31 kontaktiert die an der Unterseite der Federzunge 18 ausgebildeten Anlagestege 12 (vergleiche Darstellung gemäß Figur 2). Beim vollständigen Einschieben des Teststreifens 31 entlang der Einschubzunge 5 wird die Federzunge 18 mit ihrem daran ausgebildeten Bügelabschnitt 17 in die in Figur 4 dargestellte erste Bügellage 24 ausgelenkt. In der ersten Bügellage 24, welche die Verriegelungsposition des in Figur 4 nicht dargestellten Deckel- bzw. Klappenelementes in der Gehäuseöffnung 30 bildet, liegt die erste Seitenfläche 22 der Federzunge 18 dem ersten Anschlag 15 gegenüber.. Gleichzeitig liegt die zweite Seitenfläche 23 der Federzunge 18 dem zweiten Anschlag 16 gegenüber. Der Bügelabschnitt 17 der Federzunge 18 blockiert in der in Figur 4 dargestellten ersten Bügellage 24 eine seitliche Auslenkung der Federschenkel 13 und 14, so dass eine Entriegelung des Deckelelementes 3, d.h. dessen Entnahme aus der in Figur 3 dargestellten Gehäuseöffnung 30, sei es absichtlich oder versehentlich, bei in die Einschuböffnung 4 eingeschobenen Teststreifen 31 nicht mehr möglich ist. Dadurch wird verhindert, dass ein bei eingeschobenem Messstreifen 31 innerhalb des Schnelldiagnosegerätes 1 ablaufender Messvorgang abgebrochen wird bzw. Fehlmessungen dadurch auftreten, dass während des Messvorganges die Gehäuseöffnung 30 geöffnet wird und sich die Temperatur innerhalb des Gerätegehäuses 3 während des Messvorganges schlagartig ändert.

Aus der Darstellung gemäß Figur 4 geht darüber hinaus hervor, dass an den Aussenseiten der Federschenkel 13 und 14 jeweils ein erster Zapfen 19 sowie ein zweiter Zapfen 20 angespritzt sind. In Bezug auf die an der Innenseite der Federschenkel 13, 14 ausgebildeten, . der Federzunge 18 zuweisenden Anschläge 15 und 16 liegen der erste Zapfen 19 und der zweite Zapfen 20 an der Außenseite der Federschenkel 13 und 14 versetzt zum ersten Anschlag 15 und zum zweiten Anschlag 16. Die Federschenkel 13 bzw. 14 des als Verriegelungskörper fungierenden Federelementes 8 verlaufen in der Darstellung gemäß Figur 4 senkrecht zur Zeichenebene. Bezugszeichen 21 kennzeichnet die Bewegungsrichtung des Bügelabschnittes 17, d.h. der Vorderseite der Federzunge 18 des Federelementes 8, welche frei beweglich entsprechend ihrer Elastizität zwischen dem ersten Federschenkel 13 und dem zweiten Federschenkel 14 bewegbar ist. Durch den Grad der Kröpfung 33, in welchem die erste Federzunge 18 (vergleiche Darstellung gemäß Figur 3) ausgebildet werden kann, kann die Anstellkraft der Federzunge 18 an den in die Einschuböffnung 4 einzuschiebenden Teststreifen 31 vorgegeben werden.

In der Darstellung gemäß Figur 5 ist das als Verriegelungskörper dienende Federelement in seiner zweiten Bügellage, d.h. in einer die Freigabe des Deckel- bzw. Klappenelementes ermöglichenden Position dargestellt.

Ist in die Einschuböffnung 4 des Gerätegehäuses 2 gemäß der Darstellung in Figur 1 auf der Einschubzunge 5 kein Teststreifen 31 vorhanden, so fehlt es an einer die Federzunge 18, deren Bügelabschnitt 17 in Figur 5 dargestellt ist, in Bewegungsrichtung 21 auslenkenden Kraft. Die Anlagestege 12 an der dem Teststreifen zuweisenden Seite der Federzunge 18 bzw. der gekröpft ausbildbaren Federzunge 18 und des Bügelabschnittes 17 verbleiben in ihrer Position oberhalb der Einschubzunge 5. Da der Bügelabschnitt 17 der gekröpft ausbildbaren Federzunge 18 des Federelementes 8 nicht in Bewegungsrichtung 21 ausgelenkt wird, fährt dieser mit seinen Seitenflächen 22, 23 nicht zwischen dem ersten Anschlag 15 und dem zweiten Anschlag 16 ein. Daher können der erste Federschenkel 13 sowie der zweite Federschenkel 14 eine Auslenkung 32 in seitliche Richtung, angedeutet durch den Doppelpfeil, ausführen. Die in Figur 5 dargestellte zweite Bügellage 25 entspricht der zweiten Bügellage 25 wie sie auch in Figur 3 dargestellt ist.

Die seitliche Auslenkbewegung der Anschläge der Federschenkel 13 bzw. 14 mit den an deren Innenseite ausgebildeten Anschlägen 15 und 16 ist durch Bezugszeichen 32 gekennzeichnet. An der Außenseite des ersten Federschenkels 13 und des zweiten Federschenkels 14 sind der erste Zapfen 19 sowie der zweite Zapfen 20 ausgebildet, die bei einer seitlichen Auslenkung 32 der Federschenkel 14 und 13 aus ihren Einklippsstellen 10 bzw. 11 an der Unterseite 9 des Deckel- bzw. Klappenelementes 3 ausrastbar sind. Zur Freigabe des Deckels 3 fahren die Zapfen 19, 20 aus ihren Einklippsstellen 10 bzw. 11 zurück. Bedingt durch die dem als Federelement ausgebildeten Verriegelungskörper 8 innewohnende Federkraft, schnellen die am ersten Federschenkel 13 und am zweiten Federschenkel 14 ausgebildeten ersten und zweiten Zapfen 19, 20 wieder in ihre Ausgangsposition zurück. Das als Verriegelungskörper dienende Verriegelungskörper 8 verbleibt nach Entnahme des Deckel-Elementes 3 aus der Gehäuseöffnung 30 nicht in der in Figur 3 dargestellten Position, sondern geht mit diesem nach oben.

Die Anschläge 15 und 16 an den Innenseiten des ersten Federschenkels 13 bzw. des zweiten Federschenkels 14 des als Verriegelungskörper dienenden Federelementes 8 sind in einem Überstand 27 ausgebildet. Die Summe der Überstände 27 der ersten und zweiten Anschläge 15 und 16 samt der Breite des Verriegelungsabschnittes 17 am vorderen Ende der Federzunge 18 entsprechen im wesentlichen der Breite des in Figur 4 auf der Einschubzunge 5 eingeschobenen Teststreifens 31. Zur Erleichterung des Einschiebens des Teststreifens 31 in Einschubrichtung 26 ist der Überstand 27 in Bezug auf den erste Anschlag 15 bzw. den zweiten Anschlag 16 etwas größer bemessen, so dass ein leichtes und in einem Zug vornehmbares Einschieben eines Teststreifens 31 in die Einschuböffnung 4 an der Vorderseite 6 des Schnelldiagnosegerätes 1 möglich ist.

Das innerhalb des Schnelldiagnosegerätes eingesetzte Heizelement dient der Temperierung des Auswerte- oder Teststreifens. Durch das Deckelelement 3 wird das Heizelement gegen Verschmutzung geschützt und das Innere des Gerätegehäuses gegen Eintritt von Umgebungsluft, was die Messung eines zu temperierenden Auswerte- oder Teststreifens beeinträchtigen könnte. Handelt es sich bei dem Schnelldiagnosegerät um ein im rein optischen Verfahren arbeitendes Gerät, so kann durch das Deckelelement 3 störendes Umlicht während der Messung aus dem Gerätegehäuse des Schnelldiagnosegerätes ferngehalten werden. Die erfindungsgemäß vorgeschlagene Verriegelung durch den Test- bzw. Auswertestreifen 31 selbst, kann auch als eine solche ausgebildet werden, in welchem die Teststreifenauswertung aus elektrochemischem Wege erfolgt. Durch ein an solchen Diagnosegeräten eingesetztes Deckel- bzw. Klappenelement 3 können die entsprechenden Kontakte am Messgerät geschützt werden.

### Bezugszeichenliste

- 1: Schnelldiagnosegerät
- 2: Gerätegehäuse
- 3: Deckel-/Klappenelement
- 4: Einschuböffnung für Teststreifen
- 5: Einschubzunge
- 6: Vorderseite
- 7: Rückseite
- 8: Verriegelungskörper
- 9: Deckelunterseite
- 10: erste Einklippsstelle
- 11: zweite Einklippsstelle
- 12: Anlagestege für Teststreifen
- 13: erster Federschenkel
- 14: zweiter Federschenkel
- 15: erster Anschlag
- 16: zweiter Anschlag
- 17: Bügelabschnitt
- 18: Federzunge
- 19: erster Zapfen
- 20: zweiter Zapfen
- 21: Bewegungsrichtung Bügel
- 22: erste Seitenfläche Bügel
- 23: zweite Seitenfläche Bügel
- 24: erste Bügellage (Verriegelungsposition Deckel)
- 25: zweite Bügellage (Freigabe Deckel)
- 26: Einschubrichtung Teststreifen
- 27: Überstand

- 30: Gehäuseöffnung
- 31: Teststreifen
- 32: Bewegungsrichtung Auslenkrichtung Anschläge 15, 16
- 33: Kröpfung Federzunge

## Patentansprüche

1. Schnelldiagnosegerät mit einem Gerätegehäuse (2), an welchem eine Einschuböffnung (4) für einen Teststreifen (31) vorgesehen ist, der innerhalb des Schnelldiagnosegerätes (1) ausgewertet wird und am Gerätegehäuse (2) ein durch einen Deckel- oder Klappenelement (3) verschließbare Gehäuseöffnung (30) ausgebildet ist, wobei dem Deckel- oder Klappenelement (3) ein Verriegelungskörper (8) zugeordnet ist, **dadurch gekennzeichnet, dass** der Verriegelungskörper (8) als Federelement ausgebildet ist, welches einen Bügelabschnitt (17) umfasst, der bei in die Einschuböffnung (4) eingeschobenen Teststreifen (31) eine das Federelement verriegelnde Position (24) einnimmt.

2. Schnelldiagnosegerät gemäß Anspruch 1, **dadurch gekennzeichnet, dass** der Bügelabschnitt (17) an einer Federzunge (18) des Verriegelungskörpers (8) ausgebildet ist, die zwischen Federschenkeln (13, 14) federnd angeordnet ist.

3. Schnelldiagnosegerät gemäß Anspruch 2, **dadurch gekennzeichnet, dass** die Federzunge (18) mit einer Kröpfung (33) ausgebildet ist.

4. Schnelldiagnosegerät gemäß Anspruch 2, **dadurch gekennzeichnet, dass** an der dem Teststreifen (31) zuweisenden Seite der Federzunge (18) Anlagestege (12) verlaufen.

5. Schnelldiagnosegerät gemäß Anspruch 2, **dadurch gekennzeichnet, dass** an den Federschenkeln (13, 14) des Federelementes (8) Seitenflächen (22, 23) des Bügelabschnittes (17) gegenüberliegende Anschläge (15, 16) ausgebildet sind.

6. Schnelldiagnosegerät gemäß Anspruch 5, **dadurch gekennzeichnet, dass** der Bügelabschnitt (17) bei in die Verriegelungskörper (4) eingeführtem Teststreifen (31) zwischen die Anschläge (15, 16) gestellt ist und deren Auslenkung in Seitenrichtung (32) blockiert.

7. Schnelldiagnosegerät gemäß Anspruch 5, **dadurch gekennzeichnet, dass** der Bügelabschnitt (17) in einer zweiten Bügelabschnittlage (25) ohne den Teststreifen (31) eine Auslenkung (32) der Anschläge (15, 16) in Seitenrichtung ermöglicht und das Deckel- oder Klappenelement (3) entriegelbar ist.

8. Schnelldiagnosegerät gemäß Anspruch 2, **dadurch gekennzeichnet, dass** an den Federschenkeln (13, 14) Zapfen (19, 20) angeordnet sind, die in Einklippsstellen (10, 11,) am Deckel- oder Klappenelement (3) einführbar sind.

9. Schnelldiagnosegerät gemäß Anspruch 5, **dadurch gekennzeichnet, dass** die Anschläge (15, 16) an den Federschenkeln (13, 14) in einem Überstand (27) ausgeführt sind.

10. Schnelldiagnosegerät gemäß der Ansprüche 5 und 8, **dadurch gekennzeichnet, dass** die Zapfen (19, 20) und die Anschläge (15, 16) an den Federschenkeln (13, 14) versetzt zueinander positioniert sind.

11. Schnelldiagnosegerät gemäß Anspruch 1, **dadurch gekennzeichnet, dass** der Verriegelungskörper (8) im Deckel- oder Klappenelement (3) angeordnet ist.

12. Schnelldiagnosegerät gemäß Anspruch 1, **dadurch gekennzeichnet, dass** der Verriegelungskörper (8) im Gerätegehäuse (2) aufgenommen ist.

## Claims

1. Rapid diagnosis device having a device housing (2), on which there is provided an insertion opening (4) for a test strip (31) which is evaluated inside the rapid diagnosis device (1), and on which device housing (2) there is constructed a housing opening (30) which can be closed by a cover or flap element (3), a locking member (8) being associated with the cover or flap element (3), **characterised in that** the locking member (8) is in the form of a resilient element which comprises a curved portion (17) which takes up a position (24) locking the resilient element when test strips (31) are inserted into the insertion opening (4).

2. Rapid diagnosis device according to claim 1, **characterised in that** the curved portion (17) is constructed on a resilient tongue (18) of the locking member (8), which tongue (18) is arranged resiliently between resilient members (13, 14).

3. Rapid diagnosis device according to claim 2, **characterised in that** the resilient tongue (18) is constructed so as to have an offset portion (33).

4. Rapid diagnosis device according to claim 2, **characterised in that** abutment webs (12) extend at the side of the resilient tongue (18) directed towards the test strip (31).

5. Rapid diagnosis device according to claim 2, **characterised in that** there are constructed on the resilient members (13, 14) of the resilient element (8) stops (15, 16) which are opposite lateral faces (22, 23) of the curved portion (17).

6. Rapid diagnosis device according to claim 5, **characterised in that** the curved portion (17) is positioned between the stops (15, 16) when the test strip (31) is introduced into the locking members (4) and blocks displacement thereof in a lateral direction (32).

7. Rapid diagnosis device according to claim 5, **characterised in that** the curved portion (17) in a second curved portion position (25) without the test strip (31) allows displacement (32) of the stops (15, 16) in a lateral direction and the cover or flap element (3) can be unlocked.

8. Rapid diagnosis device according to claim 2, **characterised in that** there are arranged on the resilient members (13, 14) lugs (19, 20) which can be introduced into clip-fit locations (10, 11) on the cover or flap element (3).

9. Rapid diagnosis device according to claim 5, **characterised in that** the stops (15, 16) on the resilient members (13, 14) are constructed in a projection (27).

10. Rapid diagnosis device according to claim 5 and claim 8, **characterised in that** the lugs (19, 20) and the stops (15, 16) on the resilient members (13, 14) are positioned so as to be offset relative to each other.

11. Rapid diagnosis device according to claim 1, **characterised in that** the locking member (8) is arranged in the cover or flap element (3).

12. Rapid diagnosis device according to claim 1, **characterised in that** the locking member (8) is received in the device housing (2).

## Revendications

1. Appareil pour diagnostic rapide comportant un boîtier d'appareil (2) sur lequel est prévu un orifice d'insertion (4) pour une bande de test (31) qui est évaluée dans l'appareil pour diagnostic rapide (1) et tel qu'un orifice de boîtier (30) pouvant être fermé par un élément de couvercle ou de volet (3) est réalisé sur le boîtier d'appareil (2), un corps de verrouillage (8) étant associé à l'élément de couvercle ou de volet (3), **caractérisé en ce que** le corps de verrouillage (8) se présente sous la forme d'un élément élastique qui comprend une partie en forme d'étrier (17) qui prend une position (24) de blocage de l'élément élastique lorsque la bande de test (31) est insérée dans l'orifice d'insertion (4).

2. Appareil pour diagnostic rapide selon la revendication 1, **caractérisé en ce que** la partie en forme d'étrier (17) est réalisée sur une languette élastique (18) du corps de verrouillage (8), laquelle est disposée de manière élastique entre des branches élastiques (13, 14).

3. Appareil pour diagnostic rapide selon la revendication 2, **caractérisé en ce que** la languette élastique (18) est réalisée de manière contre-coudée (33).

4. Appareil pour diagnostic rapide selon la revendication 2, **caractérisé en ce que** des nervures d'appui (12) longent le côté de la languette élastique (18) tourné vers la bande de test (31).

5. Appareil pour diagnostic rapide selon la revendication 2, **caractérisé en ce que** des butées (15, 16) opposées à des faces latérales (22, 23) de la partie en forme d'étrier (17) sont réalisées sur les branches élastiques (13, 14) de l'élément élastique (8).

6. Appareil pour diagnostic rapide selon la revendication 5, **caractérisé en ce que** la partie en forme d'étrier (17), lorsque la bande de test (31) est introduite dans les corps de verrouillage (4), est placée entre les butées (15, 16) et bloque leur déviation en direction latérale (32).

7. Appareil pour diagnostic rapide selon la revendication 5, **caractérisé en ce que** la partie en forme d'étrier (18), dans une deuxième position de la partie en forme d'étrier (25) sans la bande de test (31), permet une déviation (32) des butées (15, 16) en direction latérale et l'élément de couvercle ou de volet (3) peut être déverrouillé.

8. Appareil pour diagnostic rapide selon la revendication 2, **caractérisé en ce que** sont situés, sur les branches élastiques (13, 14), des ergots (19, 20) qui peuvent être introduits dans des points d'encliquetage (10, 11) sur l'élément de couvercle ou de volet (3).

9. Appareil pour diagnostic rapide selon la revendication 5, **caractérisé en ce que** les butées (15, 16) sont réalisées en porte-à-faux (27) sur les branches élastiques (13, 14).

10. Appareil pour diagnostic rapide selon les revendications 5 et 8, **caractérisé en ce que** les ergots (19, 20) et les butées (15, 16) sont positionnés de manière décalée les uns par rapport aux autres sur les branches élastiques (13, 14).

11. Appareil pour diagnostic rapide selon la revendication 1, **caractérisé en ce que** le corps de verrouillage (8) est situé dans l'élément de couvercle ou de volet (3).

12. Appareil pour diagnostic rapide selon la revendication 1, **caractérisé en ce que** le corps de verrouillage (8) est logé dans le boîtier d'appareil (2).
